(19) Europäisches Patentamt
European Patent Office
Office européen des brevets

(11) Publication number: 0 325 044 A2

# (12) EUROPEAN PATENT APPLICATION

(21) Application number: 88312042.0

(22) Date of filing: 19.12.88

(51) Int. Cl.4: C07K 7/64 , A61K 37/02

The title of the invention has been amended (Guidelines for Examination in the EPO, A-III, 7.3).

(30) Priority: 22.12.87 GB 8729802

(43) Date of publication of application: 26.07.89 Bulletin 89/30

(84) Designated Contracting States: AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Wootton, Gordon Beecham Pharmaceuticals
Coldharbour Road The Pinnacles
Harlow Essex CM19 5AD(GB)
Inventor: Watts, Eric Alfred Beecham Pharmaceuticals
Coldharbour Road The Pinnacles
Harlow Essex CM19 5AD(GB)

(74) Representative: Jones, Pauline et al
Beecham Pharmaceuticals Patents & Trade Marks Dept. Great Burgh Yew Tree Bottom Road
Epsom Surrey KT18 5XQ(GB)

## (54) Pharmacologically active cyclic peptide.

(57) A pharmacologically active cyclic peptide of formula (I), or a pharmaceutically acceptable salt thereof:

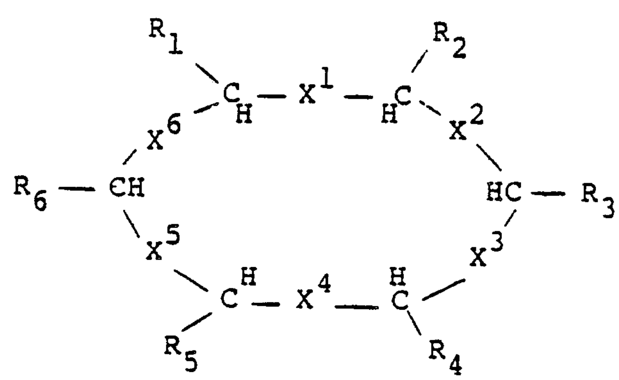

(I)

wherein
$R_1$ and $R_2$ are independently $(CH_2)_nNH_2$ or $(CH_2)_nNH\text{-}C=NH.NH_2$ wherein n is 3-6;
$R_3$ is $CH_2R_7$ wherein $R_7$ is phenyl optionally substituted by one or two substituents selected from halogen, hydroxy, $C_{1-4}$ alkyl and $C_{1-4}$ alkoxy, or $R_7$ is $C_{1-6}$ alkyl;
$R_4$, $R_5$ and $R_6$ are the same or different $C_{1-7}$ alkyl groups;
four of $X^1$ to $X^6$ are amide linkages and the other two are amide linkages or linkages wherein the amide bond is replaced by an enzyme stabilised linkage;
provided that, when $R_7$ is phenyl, at least one of the three carbon atoms depicted in formula (I) attached to $R_4$, $R_5$ and $R_6$, is in the L-configuration.

EP 0 325 044 A2

## NOVEL COMPOUNDS

This invention relates to novel cyclic peptides having pharmacological activity, to processes for their preparation and their use as pharmaceuticals.

EP-A-225020 (Beecham Group p.l.c.) describes a group of peptides comprising, in sequence, units selected from the amino acids residues 17 to 24 of VIP (vasoactive intestinal peptide) and consisting at least of the amino acid residues 18 to 23, or an analogue thereof wherein one or more of the amino acid residues is replaced by an equivalent other amino acid.

A group of novel cyclic peptides has now been discovered, based on the 18-23 amino acid residues of VIP; these cyclic peptides having pharmacological activity in reducing acid/pepsin induced duodenal damage, and therefore they are of potential use in the treatment of gastro-intestinal disorders such as peptic ulcer disease.

Accordingly, the present invention provides a pharmacologically active cyclic peptide of formula (I), or a pharmaceutically acceptable salt thereof:

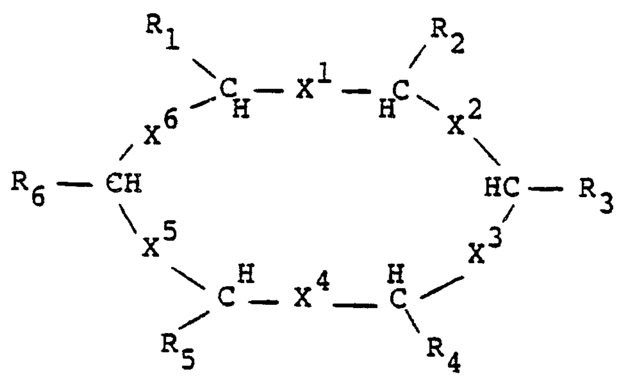

(I)

wherein

$R_1$ and $R_2$ are independently $(CH_2)_nNH_2$ or $(CH_2)_nNH\text{-}C=NH.NH_2$ wherein n is 3-6;

$R_3$ is $CH_2R_7$ wherein $R_7$ is phenyl optionally substituted by one or two substituents selected from halogen, hydroxy, $C_{1-4}$ alkyl and $C_{1-4}$ alkoxy, or $R_7$ is $C_{1-6}$ alkyl;

$R_4$, $R_5$ and $R_6$ are the same or different $C_{1-7}$ alkyl groups;

four of $X^1$ to $X^6$ are amide linkages and the other two are amide linkages or linkages wherein the amide bond is replaced by an enzyme stabilised linkage;

provided that, when $R_7$ is phenyl, at least one of the three carbon atoms depicted in formula (I) attached to $R_4$, $R_5$ and $R_6$, is in the L-configuration.

Suitable examples of $R_4$, $R_5$, $R_6$ and $R_7$ when alkyl include methyl, ethyl, n- and iso-propyl, n-, iso-, sec- and tert-butyl.

Suitable examples of substituents for $R_7$ when phenyl include hydroxy, chloro, bromo, fluoro, methyl, ethyl, methoxy and ethoxy.

Suitable examples of enzyme stabilised amide linkage replacements in $X^1$ to $X^6$ include $-CONCH_3-$ $-CSNH-$, $-CH_2NH-$, $-CH=CH-$ (cis and trans) and $-CH_2S-$. The amide bond may also be stabilised by methyl substitution at the adjacent α-carbon atom, where appropriate.

In regard to enzyme stabilisation of amide linkages, reference is hereby made to the following literature:

1. A. Spatola in 'Chemistry and Biochemistry of Amino Acids, Peptides and Proteins'; B. Weinstein (Ed), Marcel Dekker (New York) 1983, Vol. 7 pp 267-358.

2. J. Samanen in 'J. Biomedical Polypeptides'; L.G. Gebelein and L.E. Carraher Jnr. (Eds), Plenum (New York) 1985 pp 279-382.

3. D. Tourwé in 'Janssen Chim. Acta'; F. Meskens (Ed.) 1985, 3 (1), 3-15 (C.A. 1985, 103, 105267).

4. J.S. Morley in 'Trends in Pharmaceutical Sciences' 1980, p463 (publ. Elsevier).

5. V.J. Hruby in 'Life Sciences' 1982, 31, pp 189-199.

In a further aspect, the present invention provides a cyclic peptide of formula (IA), or a pharmaceutically acceptable salt thereof:

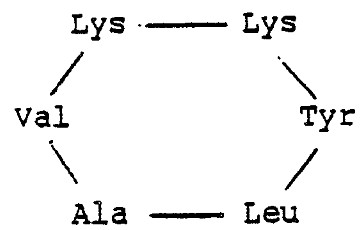

(IA)

wherein the amino acid residues are as defined below and wherein one or more of the amino acid residues may be replaced by an equivalent other amino acid.

The amino acid components may be in the D- or L- form.

When one or more of the amino acid residues is replaced by an equivalent other amino acid, these equivalent amino acids may be naturally occurring or non-naturally occurring, in the L- or in the D- or DL-form, subject to the proviso in formula (I). Preferably, the Lys-Lys-Tyr (or $R_1$, $R_2$, $R_3$ carbon atoms) are in the L-configuration. Preferably the Leu, Ala and Val (or $R_4$, $R_5$ $R_6$ carbon atoms) are in the D-configuration.

Abbreviations used for amino acids are as follows:

| Amino-acid | Abbreviation |
|---|---|
| Alanine | Ala |
| Leucine | Leu |
| Lysine | Lys |
| Norleucine | Nle |
| Phenylalanine | Phe |
| Tyrosine | Tyr |
| Valine | Val |

Amino acids can be considered as members of different classes; such groupings are well known. Replacement of an amino acid of the peptide by an equivalent amino acid may be by another amino acid of the same class, and where an amino acid can be grouped into two or more classes, replacement may be made from one or more of these classes.

Thus for example:

-alanine may be replaced by another hydrophobic amino acid, e.g. norvaline;
-valine may be replaced by another hydrophobic amino acid, e.g. leucine or isoleucine;
-tyrosine may be replaced by another hydrophobic amino acid, especially leucine or another aromatic amino acid e.g. phenylalanine (when one of Val, Ala or Leu is in the L- form);
-leucine may be replaced by another hydrophobic amino acid, e.g. valine or isoleucine.

Acid addition salts may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, trifluoroacetic acid, propionic acid, lactic acid, citric acid, tartaric acid, fumaric acid, maleic acid, succinic acid, salicylic acid or acetylsalicylic acid.

The compounds of formula (I) are preferably in pharmaceutically acceptable form. By pharmaceutically acceptable form is meant, *inter alia*, of a pharmaceutically acceptable level of purity excluding normal pharmaceutical additives *such as* diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

The invention also provides a process for the preparation of a cyclic peptide of formula (I) or a pharmaceutically acceptable salt thereof, which process comprises the cyclisation of a linear peptide of formula (II); or a salt thereof:

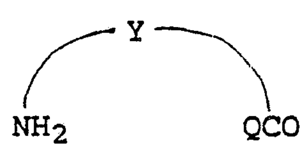

(II)

wherein Y is an optionally protected amino acid sequence corresponding to the amino acids in the desired cyclic peptide of formula (I); and Q is a leaving group; and thereafter, where necessary removing any amino acid side chain protecting groups and optionally forming a pharmaceutically acceptable salt thereof.

Q may be OH, in which case the cyclisation is carried out by conventional condensation procedures, for example using a dehydrating agent at neutral pH in an inert aprotic solvent, such as dimethylformamide (DMF) or dimethylacetamide, at a temperature of -40°C to 50°C, preferably around or below ambient temperature.

Examples of dehydrating agents include carbodiimides, such as dicyclohexylcarbodiimide (DCC) and diisopropylcarbodiimide (DIC). Alternative condensation promoting agents include carboxylic acid activating agents such as diphenylphosphoryl azide (DPPA).

The pH may be adjusted using an appropriate mild acid or base, such as $NaHCO_3$, dilute hydrochloric acid, diisopropylethylamine (DIPEA) or triethylamine.

The compound of formula (II) may be in the form of a salt, such as that with organic acids (e.g. the trifluoracetate salt) or $Bu_4N^+$ salt.

Another suitable value for Q is $N_3$, which is usually obtained from the corresponding compound of formula (II) wherein Q is replaced by $NHNH_2$ i.e. the corresponding hydrazide of formula (III):

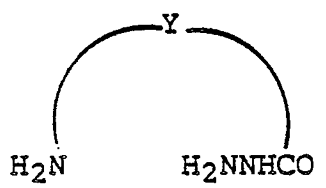

(III)

The compound of formula (III) is converted to the corresponding azide of formula (II), by reaction with nitrous acid in situ followed by neutralisation with an inorganic base, such as sodium hydrogen carbonate, or an organic base, such as triethylamine or diisopropylethylamine.

Reagents which generate nitrous acid include alkylnitrites, such as t-butyl nitrite or isoamyl nitrite or alkali metal nitrite salt, such as sodium nitrite or potassium nitrite, in the presence of a strong acid such as hydrochloric, sulphuric or, if desired or necessary, phosphoric acid. The reaction is carried out in non aqueous solvents when an alkyl nitrite is used, or in an aqueous/non aqueous mix of solvents when an alkali metal nitrite salt is used. Non aqueous solvents include, dioxane, chloroform, ethyl acetate or dichloromethane. Suitable aqueous/non aqueous solvent mixtures include water with dimethylformamide (DMF), tetrahydrofuran (THF) or dioxane.

The azide of formula (II) cyclises to the desired compound of formula (I) under the above conditions.

The selection of side chain protecting groups is, dictated by a number of factors, such as the particular coupling conditions, the amino acid components involved in the stepwise synthesis and the choice of functionalised resin.

Amino protecting groups ordinarily employed include those which are well known in the art, for example, urethane protecting substituents such as benzyloxycarbonyl (carbobenzoxy), (CBZ = Z), t-butyloxycarbonyl (Boc) and 9-Fluorenylmethyloxy carbonyl (Fmoc). It is preferred to utilise the Fmoc group for protecting the α-amino group in the amino acids undergoing reaction at the carboxylic end of the said amino acid. The carboxylic end is activated by formation of an anhydride, (from 2 mole- equivalent of protected amino acid and DCC or DIC in a solvent such as THF or $CH_2Cl_2$), or by formation of an activated ester such as a substituted phenyl ester, e.g. ortho-or para-nitrophenyl or penta-fluorphenyl. The activated ester can be formed in situ via equimolar amounts of DCC or DIC and the appropriate alcohol.

The side chain amino group of Lysine can be protected by the Boc group or CBZ or 2,6-Cl-CBZ. The OH of Tryosine can be protected by a t-butyl (Bu) group or 2,6-chlorobenzyl group. The choice of protecting groups will depend on the final method of deprotection, for example Boc and Bu groups can be removed by trifluoroacetic acid. It might however, be preferable to ensure no loss of side chain protecting groups during synthesis of the linear peptide and choose the more acid stable CBZ and 2,6-Cl-Bz groups which can be removed by hydrogenolytic methods.

Deprotection takes place conventionally. For example, prolonged treatment with strong acids such as trifluoracetic acid (TFA), trifluoromethane-sulphonic acid (TFMSA) or HF will generate the corresponding salts of the required cyclic peptide. Alternatively, milder deprotection can be achieved by hydrogenolytic methods where the choice of final salt can be a function of the solvent used, e.g. 5% Pd on C in ethanol:water:acetic acid 8:2:1 will yield the acetate salt of the final product.

Purification of the product may take place by conventional methods, as described in the Examples hereinafter.

The linear peptide intermediate of formula (II) is prepared in accordance with the methods generally described in EP-A-225020, the subject matter of which is incorporated herein by reference.

It is generally not critical which amino acid is selected to be at the C terminal position provided that the sequence of amino acids is the desired sequence required for the cyclised peptide.

The selection of the first amino acid to start the chain is not critical, since the linear peptide will be cyclised, but there may be other factors which may prefer one starting amino acid over another. For example, the choice of side chain protecting groups may influence the choice of N terminus amino acid, the ability to selectively remove the N terminus, and the desired sequence of amino acids in the cyclic peptide. Other factors include the susceptibility of the amino acids to racemisation (i.e. it is preferred that the first amino acid is not susceptible), and steric considerations which could affect the cyclisation of the compound of formula (III).

Synthesis of the linear peptide by solid phase is conducted in a stepwise manner on a functionalised resin. The nature of the functionalisation is chosen such that the linear peptide can be removed in such a way that the side chain protecting groups remain intact and the linear peptide only has exposed the N terminus amino moiety and C terminus carboxylic acid moiety.

It is generally considered to be good practice, although not absolutely necessary, to incorporate a reference amino acid within the functionalisation so that the synthesis can be monitored at any time by amino acid analysis. A suitable spacing group can be incorporated to ease synthetic attachment of the reference amino acid and a 'linker' moiety. It is the chemical integrity of this 'linker' that enables the user to build the linear peptide in a sequential fashion with suitable derivatised amino acids that allow (i) attachment of the first amino acid to the linker (usually with an ester link) (ii) selective removal of N terminal protection of this first amino acid to enable sequential build up of the linear peptide (iii) cleavage of the linear peptide to give the linear peptide, as the N terminus amino-C terminus carboxylic acid, with intact side chain protection.

The eventual choice of the resin may be influenced by the choice of the functionalising group, the length of linear peptide, whether the synthesis is carried out in a manual mode, automatic or semi-automatic mode. The resin itself may be supported or exist in a free bead form and can swell in a predetermined solvent.

The pre-peptide resin can thus take the form as illustrated below:

LINKER...REFERENCE AMINO-ACID..SPACER..RESIN

Possible combinations are:

A

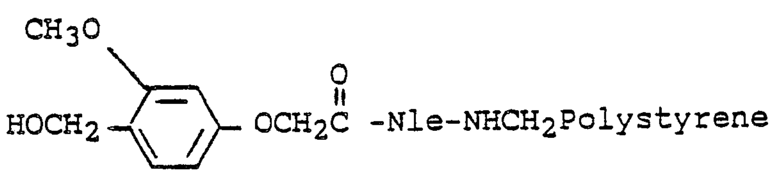

B 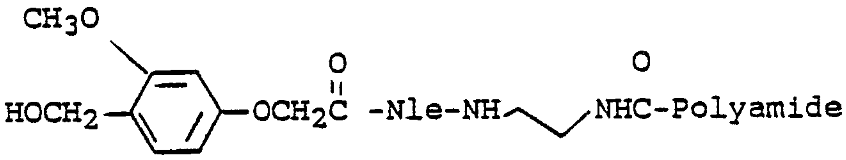

C 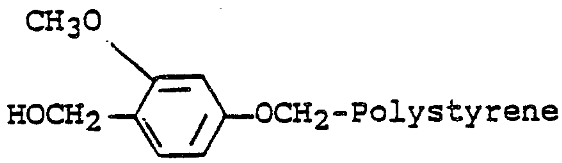

D 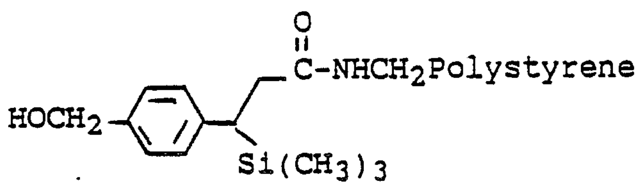

Cleavage from A, B or C occurs with mild acid, for example 1-5% trifluoracetic acid in dichloromethane, or from D with $Bu_4N^+F^-$ to give the linear peptide of formula (II) as the TFA salt or $Bu_4N^+$ salt respectively.

The above linkers are known, for example, linkers A, B and C are described by R.C. Sheppard and B.J. Williams in Int. J. Peptide Res. 20, 1982, 451-454.

It is preferred, on a large scale however, that the linear peptide intermediate of formula (II) is prepared by classical solution methods. When intermediates comprising enzyme stabilised amide bonds are required, reference is hereby made to the appropriate references 1. - 5. as listed hereinbefore.

The present invention further provides a cyclic peptide of the present invention, for use in a method of treatment of the human or animal body.

Where the cyclic peptide is in the form of a salt thereof, it should of course be understood that this is a physiologically tolerable salt, which is pharmaceutically acceptable.

The cyclic peptide of the invention may be administered per se or, preferably, as a pharmaceutical composition also including a pharmaceutically acceptable carrier.

Accordingly, the present invention provides a pharmaceutical composition, which comprises a cyclic peptide of the present invention, in admixture or conjunction with a pharmaceutically acceptable carrier.

The preparation may, if desired, be in the form of a pack accompanied by written or printed instructions for use.

In accordance with conventional pharmaceutical practice the carrier may comprise a diluent, filler, disintegrant, wetting agent, lubricant, colourant, flavourant or other conventional additive.

Preferably, a pharmaceutical composition of the invention is in unit dosage form.

The suitable dosage range for compounds of the invention may vary from compound to compound and may depend on the condition to be treated. It will also depend, inter alia, on the relation of potency to absorbability and on the mode of administration chosen.

The compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before

use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing.

Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The present invention further provides a method of treatment of mammals, including humans, which comprises administering an effective, non-toxic, amount of a cyclic peptide of the present invention to the mammal; and a peptide or analogue of the present invention for use as a pharmaceutical, in particular for use in the treatment of peptic ulcer disease.

Conveniently, the active ingredient may be administered as a pharmaceutical composition hereinbefore defined, and this forms a particular aspect of the present invention.

A suitable dose is, for example, in the range of from 0.01 to 100, more usually 0.01 to 10, preferably 0.01 to 2.5 mg/kg. A possible daily dose for humans is, for example, 1.0 to 50 mg by intravenous infusion, 1.0 to 250 mg by aerosol or 1.0 to 500 mg by oral administration, including enteric coated capsule form.

No adverse toxicological effects are indicated at the aforementioned dosage ranges.

The following examples illustrate the invention; the following descriptions illustrate the preparation of functionalised resins.

Each cyclic peptide example was characterised by 24h acidolytic cleavage and PITC (phenylisothiocyanate) derivatised amino acid analysis (Waters Picotag System) and fast atom bombardment (FAB) mass spectrometry (Jeol D x 303).

In the following, the various protecting groups, reagents and solvents are referred to by abbreviations for convenience.

| Abbreviation | Group/Reagent etc. |
|---|---|
| Boc | t-Butyloxycarbonyl |
| Bu | t-Butyl |
| CBZ or Z | Benzyloxycarbonyl |
| Fmoc | 9-Fluorenylmethyloxycarbonyl |
| HOBT | Hydroxybenzotriazole |
| OPFP | Pentafluorophenyl ester |
| ONP | Para-nitrophenyl ester |
| DCC | Dicyclohexylcarbodiimide |
| DIC | Diisopropylcarbodiimide |
| DPPA | Diphenylphosphoryl azide |
| TFA | Trifluoroacetic acid |
| DMAP | Dimethylaminopyridine |
| DIPEA | Diisopropylethylamine |
| TFMSA | Trifluoromethanesulphonic acid |
| DCU | Dicyclohexylurea |
| DMF | Dimethylformamide |
| THF | Tetrahydrofuran |
| DMA | Dimethylacetamide |
| DMSO | Dimethylsulphoxide |

Preparation of Functionalised Resin A

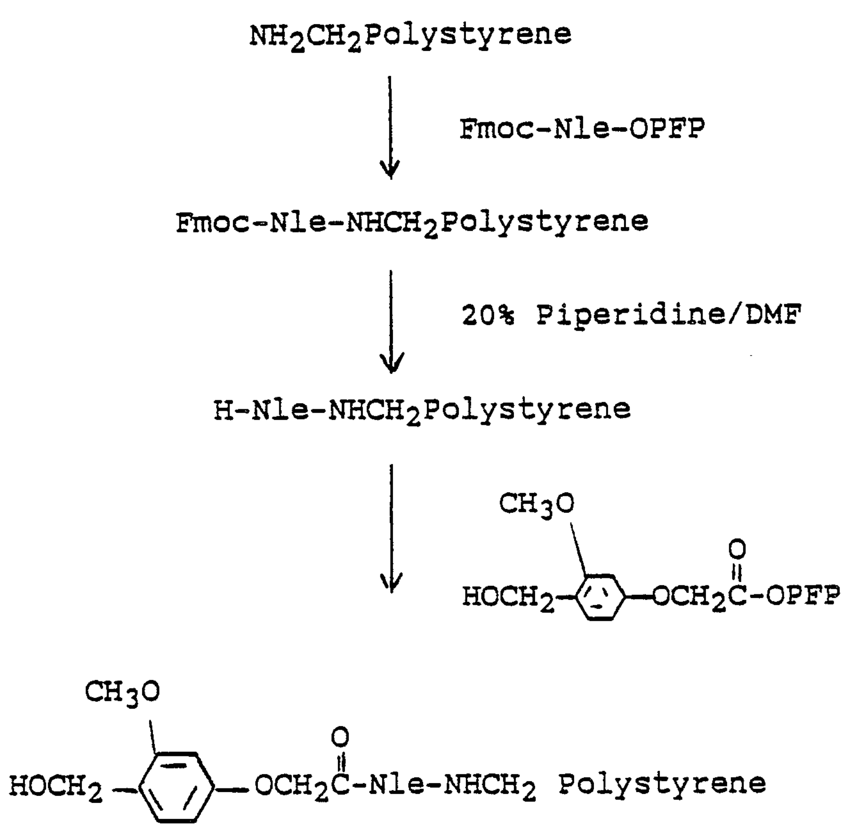


Aminomethylpolystyrene (Bachem UK Ltd) (1g, 0.5 mequiv/g.) was swollen by agitation under a continuous stream of nitrogen bubbles in 10% DMF/$CH_2Cl_2$ (20ml) for 2 x 15 minutes. Fmoc-Nle-OPFP (649mg, 2.5 equivs) and HOBT (169mg) in 10% DMF/$CH_2Cl_2$ (10-15ml) were added to the drained resin and the whole agitated for 1 hour. The reaction was monitored to completion by the Kaiser ninhydrin test

(Kaiser, E. et al. Anal. Biochem (1970) 34, 595). The resin was drained and repeatedly washed under similar agitation with DMF (10 x 15ml x 1 min). 20% Piperidine in DMF (15ml) was added to the drained resin and the mixture agitated as above for 3 minutes, and then again for 7 minutes with an intermediate DMF (2 x 15ml x 1 min) wash. The resin was then subjected to a further DMF (10 x 15ml x 1 min) agitated wash. Pentafluorophenyl 4-hydroxymethyl-3-methoxyphenoxy acetate (567mg, 3 equivs) and HOBT (202mg, 3 equivs) in DMF (10-15ml) were added to the drained resin, and the whole agitated as before for 1 hour. A wash with DMF (10 x 15ml x 1 min) gave the swollen resin ready for immediate use in the preparation of linear peptide C-terminal acids with intact side-chain protection.

This functionalised resin can alternatively be washed successively with $CH_2Cl_2$ (10 x 15ml x 1 min) and anhydrous $Et_2O$ (10 x 15ml x 1 min), drained, dried in vacuo and stored for later use.

Preparation of Functionalised Resin B

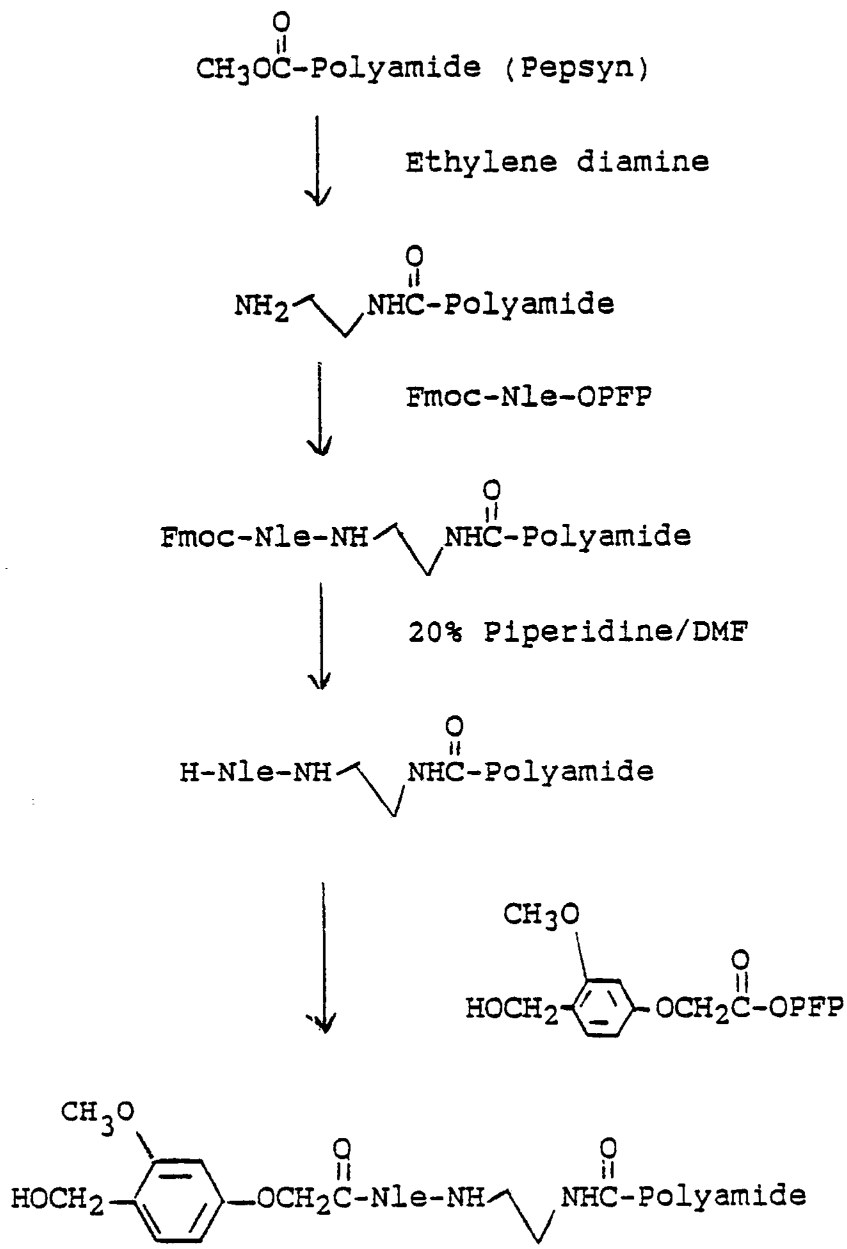


Dry Pepsyn (Cambridge Research Biochemicals Ltd) (1.0g, 0.3 mequiv.) was swollen by agitation overnight under a continuous stream of nitrogen bubbles in ethylene diamine (35-40ml). The resin was

drained and repeatedly washed under similar agitation with DMF (10 x 20ml x 1 min), 10% DIPEA in DMF (2 x 20ml x 1 min) and DMF (5 x 20ml x 1 min). Fmoc-Nle-OPFP (935mg, 6 equivs) and HOBT (243mg) in DMF (15-20ml) were added to the drained resin and the whole agitated for 1 hour. The reaction was monitored to completion by the Kaiser-ninhydrin test. The resin was drained and repeatedly washed under similar agitation with DMF (10 x 20ml x 1 min). 20% Piperidine in DMF (20ml) was added to the drained resin and agitated as above for 3 minutes, and then again for 7 minutes with an intermediate DMF (2 X 20ml x 1 min) wash. The resin was then subjected to a further DMF (10 x 20ml x 1 min) agitated wash.

Pentafluorophenyl 4-hydroxymethyl-3-methoxyphenoxy-acetate (681mg, 6 equivs) and HOBT (243mg) in DMF (20ml) was added to the drained resin and the whole agitated as before for 1 hour. The reaction was monitored as before with the Kaiser test.

A wash with DMF (10 x 20ml x 1 min) gave the swollen resin suitable for immediate preparation of the peptide C-terminal acid with intact side-chain protection.

Alternatively this functionalised resin can be washed successively with $CH_2Cl_2$ (10 x 20ml x 1 min), anhydrous $Et_2O$ (10 x 20ml x 1 min), drained, dried in vacuo and stored for later use.

[This preparation differs from that suggested by Cambridge Research Biochemicals in that it uses HOBT to increase the rate of acylation by the pentafluorophenyl esters].

Example 1

Cyclo-[D-Alanyl-L-Valyl-L-Lysyl-L-Lysyl-L-Tyrosyl-L-Leucine]

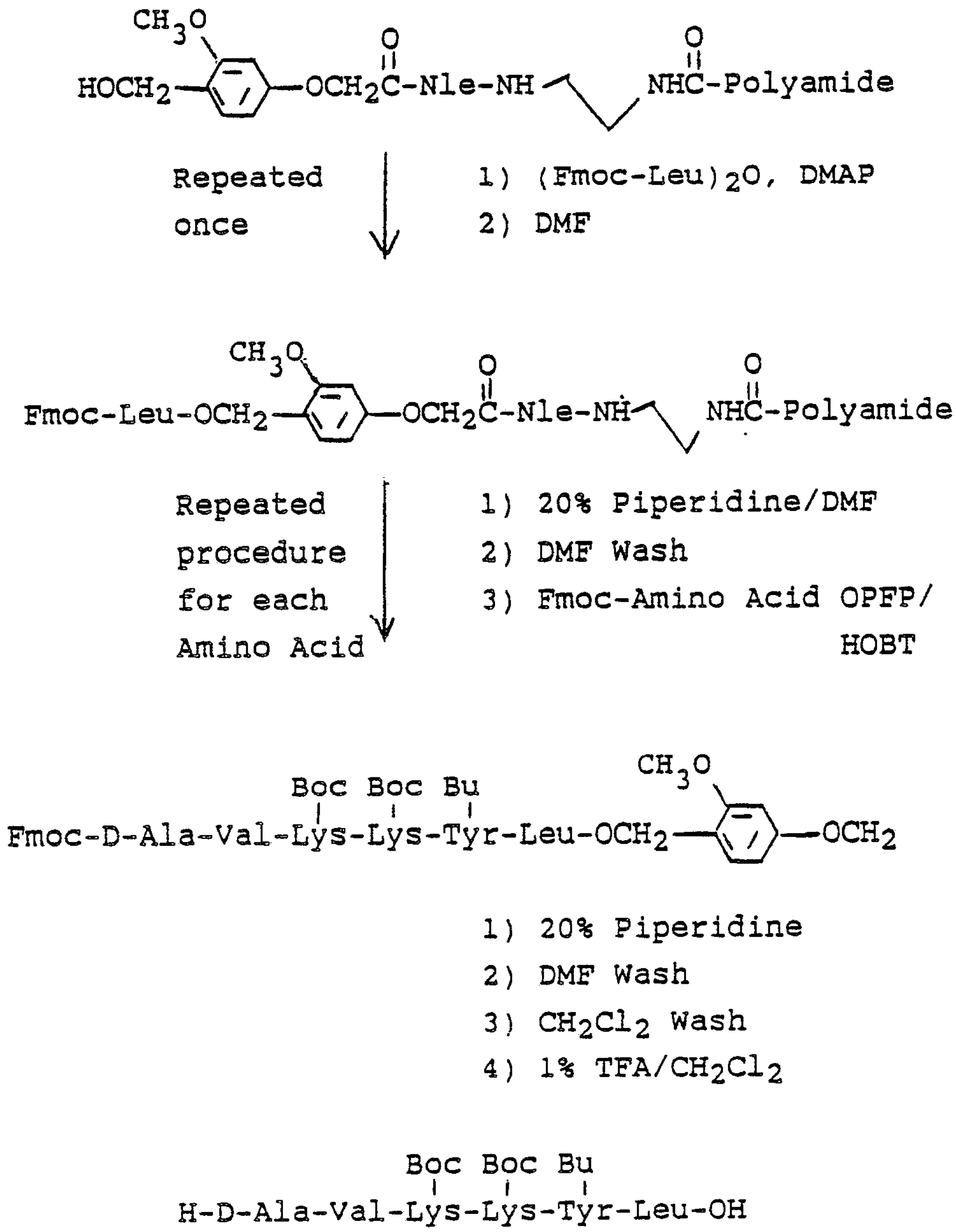

Repeated
once
1) (Fmoc-Leu)2O, DMAP
2) DMF
Repeated
procedure
for each
Amino Acid
1) 20% Piperidine/DMF
2) DMF Wash
3) Fmoc-Amino Acid OPFP/
HOBT
1) 20% Piperidine
2) DMF Wash
3) CH2Cl2 Wash
4) 1% TFA/CH2Cl2

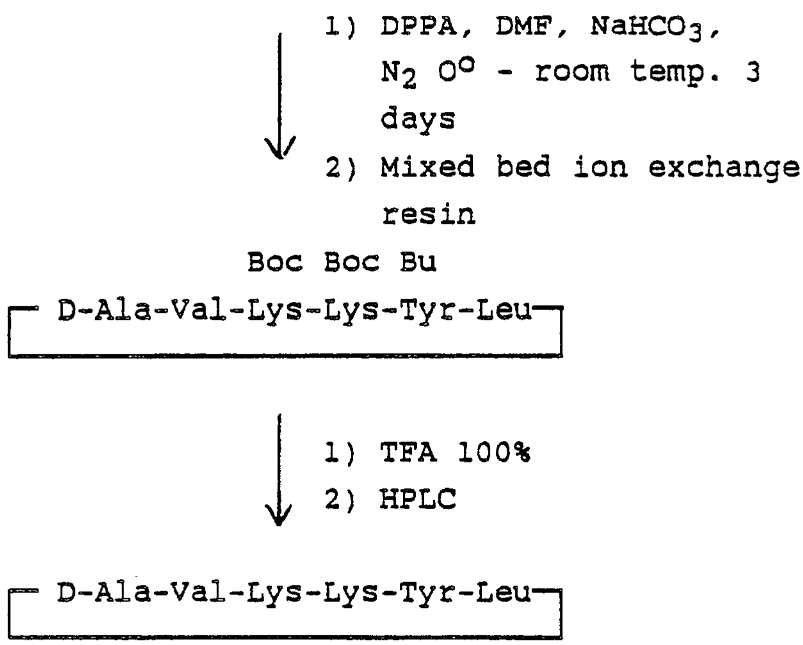


(E1)

Functionalised resin B (1g, 0.5meq/g) was prepared as outlined above, or swollen from the dried state in DMF (2 x 20ml x 15 min).

Fmoc-Leu-OH (530mg, 5 equiv) was dissolved in $CH_2Cl_2$ (4ml) with DMF (0.5ml), cooled to 0°C and treated with DIC (96mg, 0.12ml). The stirred mixture was allowed to reach room temperature over 20-25 min and evaporated in vacuo. The residue was dissolved in DMF (5-10ml) and added to the drained resin, followed by DMAP (9mg). The resin was agitated under a continuous stream of nitrogen for 1h, drained, washed with DMF (5 x 20ml x 1 min). This addition of the first amino acid was repeated, and the resin drained and washed with DMF (10 x 20 ml x 1 min).

20% Piperidine in DMF was added to the drained resin and the mixture agitated as before for 3 minutes, and then again for 7 minutes, with an intermediate wash with DMF (2 x 15ml x 1 min). The resin was then subjected to a further wash cycle with DMF (10 x 15ml x 1 min) to give the resin with the N terminus deprotected first amino-acid (Leu) attached. Amino acids (listed below) were then added to the resin in a stepwise fashion with piperidine deprotection as described above after each coupling.

In each case HOBT (122mg) was added and each coupling left for 60 minutes. Completion of reaction was monitored by the Kaiser-ninhydrin test.

| | |
|---|---|
| Fmoc-Tyr(Bu)-OPFP | 563mg |
| Fmoc-Lys(Boc)-OPFP | 571mg |
| Fmoc-Lys(Boc)-OPFP | 571mg |
| Fmoc-Val-OPFP | 455mg |
| Fmoc-D-Ala-OPFP | 430mg |

When the final amino acid was added the N-terminus was deprotected as before, drained, washed with DMF (10 x 15ml x 1 min) , $CH_2Cl_2$ (10 x 15ml x 1 min), and $Et_2O$ (10 x 15ml x 1 min) and dried in vacuo for 2 hours.

The resin was reswollen in $CH_2Cl_2$ (2 x 15 ml x 1 min), drained and treated with 1% TFA in $CH_2Cl_2$ (10 x 20ml x 5 mins). The solutions from this treatment were evaporated in vacuo and the residue treated with ether to yield impure side-chain protected amino-peptide-acid (153mg) FAB M+1 = 977 used without further purification.

This material (153mg) was dissolved in degassed DMF (200ml) and cooled to 0°C. The solution was treated with DPPA (42µL) and solid $NaHCO_3$ (59mg) and left to stir under nitrogen for 3 days reaching ambient temperature in the first 6 hours. Water (5ml) and Bio-Rad AG 501-X8 mixed bed ion exchange resin (3g) were added and the mixture stirred for 3 hours. The mixture was filtered and the insolubles washed with DMF (10-20ml). The filtrate was evaporated in vacuo and the residue treated with ether to yield impure cyclic side chain protected peptide (54mg) FAB $M+Na^+$ = 981.

A solution of this material (54mg) in TFA (10ml) at 0°C was left to stir for 4 hours under nitrogen reaching ambient temperatures over $\frac{1}{2}$ h. The solution was evaporated in vacuo, azeotroped with toluene (ca. 10ml), then ether (2 x 20ml) to yield a gummy solid. This solid was dissolved in water and lyophilised to a white solid (52mg). FAB mass spectrometry revealed the presence of required cyclic peptide, M+1 = 703 and uncyclised peptide M+1 = 721.

This mixture was subjected to repeated HPLC on µBondpak C18 reversed-phase column using 20% MeOH:80% $H_2O$ containing 0.1% TFA as eluant to yield the title compound (10mg) as the trifluoroacetate salt. FAB M+1 = 703.

## Example 2

### Cyclo-[D-Alanyl-D-Valyl-L-Lysyl-L-Lysyl-L-Tyrosyl-D-Leucine]

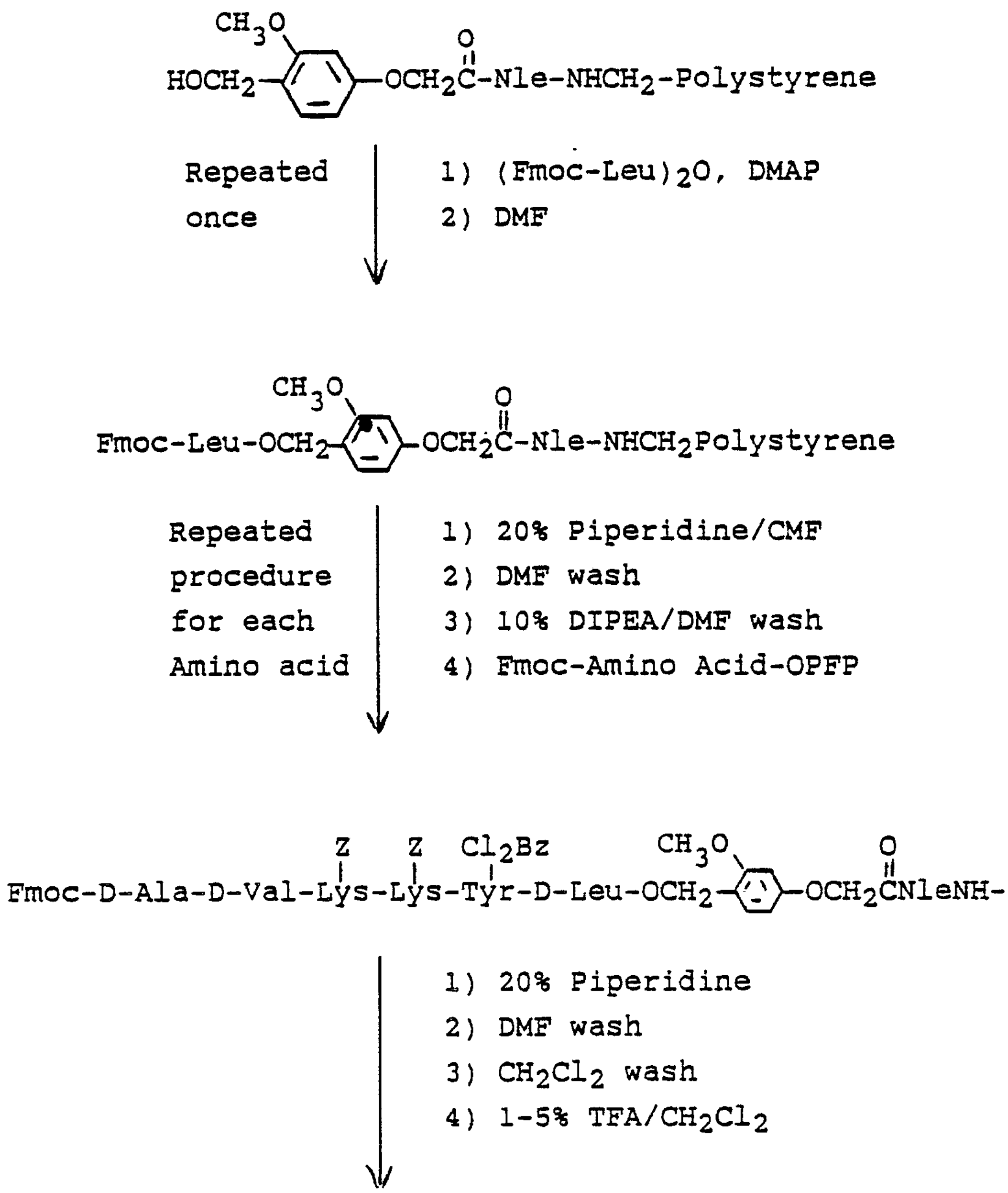
Repeated
once
1) (Fmoc-Leu)2O, DMAP
2) DMF
Repeated
procedure
for each
Amino acid
1) 20% Piperidine/CMF
2) DMF wash
3) 10% DIPEA/DMF wash
4) Fmoc-Amino Acid-OPFP
1) 20% Piperidine
2) DMF wash
3) CH2Cl2 wash
4) 1-5% TFA/CH2Cl2

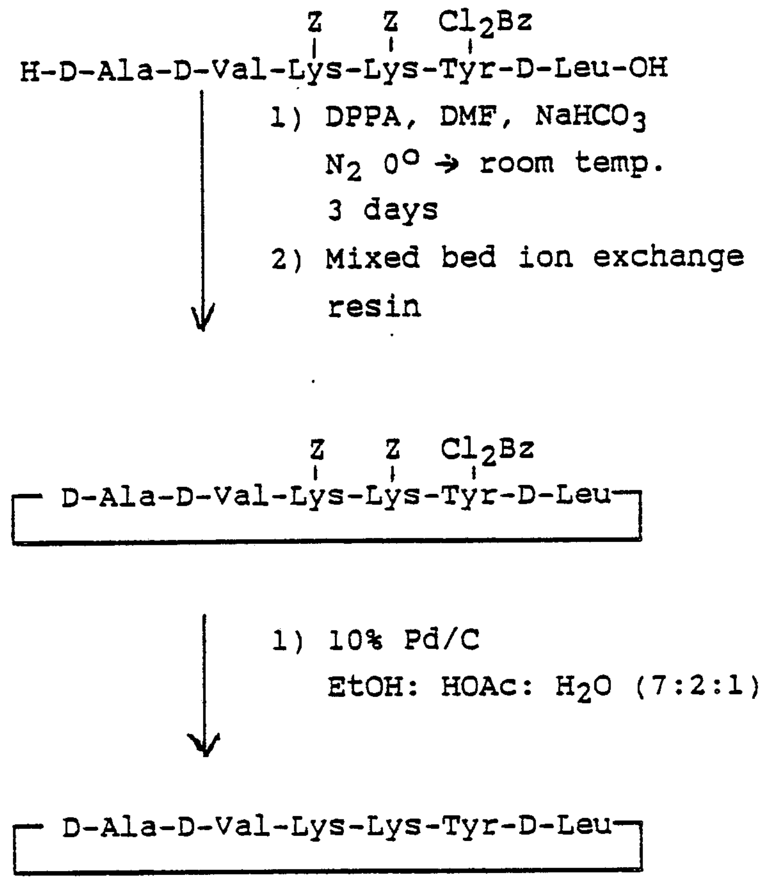


(E2)

Functionalised resin A (1g, 0.5meq/g) was prepared as outlined above, or swollen from the dried state in DMF (2 x 20ml x 15 min).

In a repeating cycle of coupling and deprotection the following amino acids were added to the resin as outlined above.

$(Fmoc\text{-}D\text{-}Leu)_2O$ (from Fmoc-Leu-OH (882mg; 5 equiv)
DIC (0.2ml) DMAP (15mg)

| | |
|---|---|
| Fmoc-Tyr $(Cl_2Bz)$-OPFP | (909mg; 2.5 equiv) |
| Fmoc-Lys-(CBZ)-OPFP | (835mg; 2.5 equiv) |
| Fmoc-Lys-(CBZ)-OPFP | (835mg; 2.5 equiv) |
| Fmoc-D-Val-OPFP | (632mg; 2.5 equiv) |
| Fmoc-D-Ala-OPFP | (597mg; 2.5 equiv) |

After the last deprotection the peptide-resin matrix was dried for at least 2 hours then swollen in $CH_2Cl_2$ and treated with 1%-5% TFA as outlined in Example 1 to yield the side chain protected linear hexapeptide (523mg; 92%) FAB M+1 = 1147 Amino Acid analysis Found Ala = 1.0; Val = 0.9; Lys = 1.9; Tyr = 1.1; Leu = 1.0.

Cyclisation of the above in like manner to that described in Example 1 gave the side-chain protected cyclic-hexapeptide (419mg; 86%) FAB M+1 = 1129 Amino acid analysis Found Ala = 0.9; Val = 0.9; Lys = 2.1; Tyr = 1.0; Leu = 1.0.

The side chain protected cyclic hexapeptide (160mg, 0.14 mmole) was dissolved in anhydrous DMF (30ml) and hydrogenolysed over 10% Pd/C (160mg) for 3 hours at ambient temperatures. The catalyst was removed by filtration through kieselguhr, and the filtrate evaporated in vacuo. The residue was taken up in 5% aqueous acetic acid (200ml), filtered and lyophilised to yield the title compound (114mg; 98%) FAB M+1 = 703, as the acetate salt.

Further purification was achieved by ion exchange chromatography on CM TriSacryl (Trade Mark) followed by HPLC assay (µBondapak via 30% MeOH: 50mM $NH_4OAc$ aq.: 0.05% HOAc).

The following examples were prepared in a similar manner:

## Example 3

Cyclo-[D-Alanyl-L-Valyl-L-Lysyl-L-Lysyl-L-Phenylalanyl-L-Leucine]acetate

FAB M+1 687

## Example 4

Cyclo-[D-Alanyl-D-Valyl-L-Lysyl-L-Lysyl-L-Leucyl-D-Leucine]acetate

FAB M+1 = 653

## Pharmacological Data

### Method for Acid/Pepsin induced duodenal damage

#### Preparation

Male Wistar rats, 180 to 300g body weight, are anaesthetised with urethane (1.75g/kg i.m.). The trachea is cannulated to ensure a clear airway, and a jugular vein is cannulated. A laparotomy is performed and a titanium fistular (3cm x 0.5cm i.d.) is inserted into the non-glandular forestomach. This is exteriorised via a stab wound in the body wall. The duodenal perfusion cannula is constructed from an 8fg oral dosing tube. This cannula has two ports situated 1cm apart on opposite sides of the cannula approximately 5cm from the proximal end of the cannula. The lumen is occluded between the two ports, and locating collars positioned proximal to the upper port and distal to the lower port. This cannula is passed via the gastric fistula through the pylorus into the proximal duodenum, and leaves the duodenum via a small incision just proximal to the Papilla of Vater. The cannula is positioned so that the proximal port is directed to the greater curvature of the duodenum, and ligated in place around the locating collars so as to produce a proximal duodenal chamber excluding gastric, biliary and pancreatic secretions.

#### Experimental Procedure

After preparation 0.9% NaCl at pH 6.5 is perfused through the duodenal chamber at 0.1ml/min for 0.5h, at which point the perfusing medium is changed to 0.9% NaCl containing 30mM HCl and 250mcg/ml pepsin (Bovine), which is perfused at the same rate for 4.5h. The animals are killed, the duodena removed, opened under 0.9% NaCl and examined for damage under a low power microscope by an observer unaware of the dosing regime using a subjective scoring system of 0 to 10. A group size of 6 is used, and significance is determined using the Mann-Whitney 'U' test. Compounds are administered in sterile saline pH 6.5 as an i.v. infusion via the jugular vein at a rate of 1.2ml/h throughout the experiment from the start of the pH 6.5 duodenal perfusion.

Using this protocol, Compound El at a dose of 450nmol/kg/h gave a 51% reduction in duodenal

damage, and compound E2 at a dose of 50nmol/kg/h gave a 45% reduction in duodenal damage. ($p<0.01$ $>0.001$).

## Claims

1. A pharmacologically active cyclic peptide of formula (I), or a pharmaceutically acceptable salt thereof:

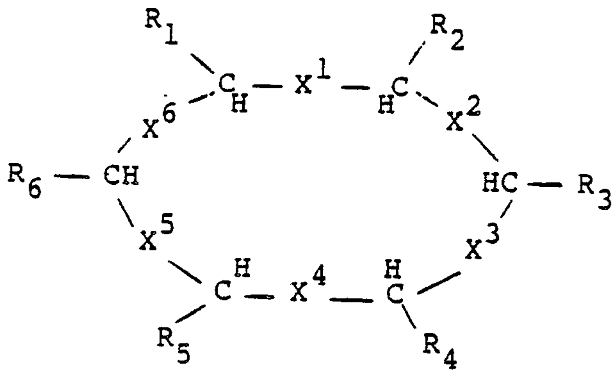

(I)

wherein

$R_1$ and $R_2$ are independently $(CH_2)_nNH_2$ or $(CH_2)_nNH\text{-}C=NH.NH_2$ wherein n is 3-6;
$R_3$ is $CH_2R_7$ wherein $R_7$ is phenyl optionally substituted by one or two substituents selected from halogen, hydroxy, $C_{1-4}$ alkyl and $C_{1-4}$ alkoxy, or $R_7$ is $C_{1-6}$ alkyl;
$R_4$, $R_5$ and $R_6$ are the same or different $C_{1-7}$ alkyl groups;
four of $X^1$ to $X^6$ are amide linkages and the other two are amide linkages or linkages wherein the amide bond is replaced by an enzyme stabilised linkage;
provided that, when $R_7$ is phenyl, at least one of the three carbon atoms depicted in formula (I) attached to $R_4$, $R_5$ and $R_6$, is in the L-configuration.

2. A cyclic peptide according to claim 1 of the formula (IA), or a pharmaceutically acceptable salt thereof:

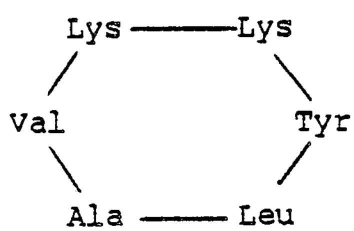

(IA)

wherein one or more of the amino acids may be replaced by an equivalent other amino acid.

3. A cyclic peptide according to claim 2 wherein Tyr is replaced by another hydrophopic amino acid, such as leucine or phenylalanine (when one of Val, Ala or Leu is in the L-form).

4. A cyclic peptide according to any one of claims 1 to 3 wherein the Lys-Lys-Tyr (or $R_1$, $R_2$, $R_3$ carbon atoms) are in the L-configuration and the Leu-Ala-Val (or $R_4$, $R_5$, $R_6$ carbon atoms) are in the D-configuration.

5. Cyclo-[D-Alanyl-L-Valyl-L-Lysyl-L-Lysyl-L-Tyrosyl-L-Leucine],
cyclo-[D-Alanyl-D-Valyl-L-Lysyl-L-Lysyl-L-Tyrosyl-D-Leucine],
cyclo-[D-Alanyl-L-Valyl-L-Lysyl-L-Lysyl-L-Phenylalanyl-L-Leucine],
cyclo-[D-Alanyl-D-Valyl-L-Lysyl-L-Lysyl-L-Leucyl-D-Leucine],
or a pharmaceutically acceptable salt of any of the foregoing.

6. A process for the preparation of a cyclic peptide according to any one of claims 1 to 5, which process comprises the cyclisation of a linear peptide of formula (II); or a salt thereof:

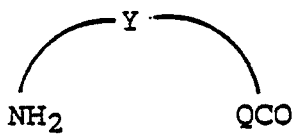

(II)

wherein Y is an optionally protected amino acid sequence corresponding to the amino acids in the desired cyclic peptide of formula (I); and Q is a leaving group; and thereafter, where necessary removing any amino acid side chain protecting groups and optionally forming a pharmaceutically acceptable salt thereof.

7. A pharmaceutical composition comprising a cyclic peptide according to any one of claims 1 to 5, and a pharmaceutically acceptable carrier.

8. A cyclic peptide according to any one of claims 1 to 5, for use as an active therapeutic substance.

9. A cyclic peptide according to any one of claims 1 to 5, for use in the treatment of peptic ulcer disease.

10. Use of a cyclic peptide according to any one of claims 1 to 5 in the manufacture of a medicament for use in the treatment of peptic ulcer disease.

Claims for the following Contracting State: ES

1. A process for the preparation of a pharmacologically active cyclic peptide of formula (I), or a pharmaceutically acceptable salt thereof:

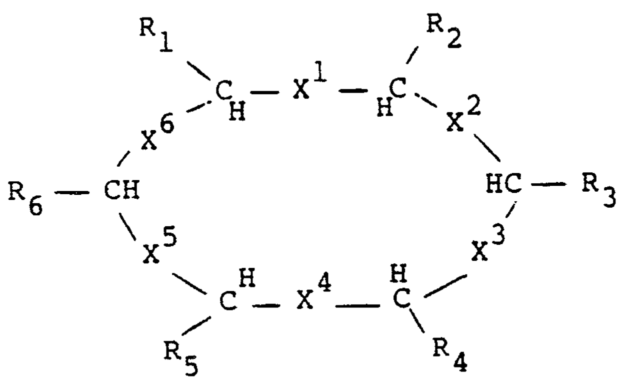

(I)

wherein

$R_1$ and $R_2$ are independently $(CH_2)_nNH_2$ or $(CH_2)_nNH\text{-}C=NH.NH_2$ wherein n is 3-6;

$R_3$ is $CH_2R_7$ wherein $R_7$ is phenyl optionally substituted by one or two substituents selected from halogen, hydroxy, $C_{1-4}$ alkyl and $C_{1-4}$ alkoxy, or $R_7$ is $C_{1-6}$ alkyl;

$R_4$, $R_5$ and $R_6$ are the same or different $C_{1-7}$ alkyl groups;

four of $X^1$ to $X^6$ are amide linkages and the other two are amide linkages or linkages wherein the amide bond is replaced by an enzyme stabilised linkage;

provided that, when $R_7$ is phenyl, at least one of the three carbon atoms depicted in formula (I) attached to $R_4$, $R_5$ and $R_6$, is in the L-configuration; which process comprises the cyclisation of a linear peptide of formula (II); or a salt thereof:

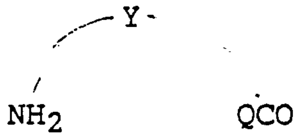

(II)

wherein Y is an optionally protected amino acid sequence corresponding to the amino acids in the desired cyclic peptide of formula (I); and Q is a leaving group; and thereafter, where necessary removing any amino acid side chain protecting groups and optionally forming a pharmaceutically acceptable salt thereof.

2. A process according to claim 1 for the preparation of a cyclic peptide according to claim 1 of the formula (IA), or a pharmaceutically acceptable salt thereof:

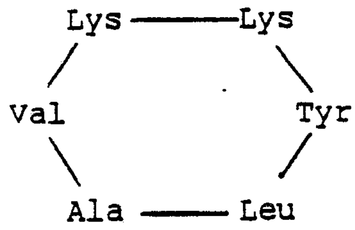

(IA)

wherein one or more of the amino acids may be replaced by an equivalent other amino acid.

3. A process according to claim 2 for the preparation of cyclic peptide of formula (IA) wherein Tyr is replaced by another hydrophopic amino acid, such as leucine or phenylalanine (when one of Val, Ala or Leu is in the L-form).

4. A process according to any one of claims 1 to 3 for the preparation of a cyclic peptide wherein the Lys-Lys-Tyr (or $R_1$, $R_2$, $R_3$ carbon atoms) are in the L-configuration and the Leu-Ala-Val (or $R_4$, $R_5$ $R_6$ carbon atoms) are in the D-configuration.

5. A process according to claim 1 for the preparation of cyclo-[D-Alanyl-L-Valyl-L-Lysyl-L-Lysyl-L-Tyrosyl-L-Leucine],
cyclo-[D-Alanyl-D-Valyl-L-Lysyl-L-Lysyl-L-Tyrosyl-D-Leucine],
cyclo-[D-Alanyl-L-Valyl-L-Lysyl-L-Lysyl-L-Phenylalanyl-L-Leucine],
cyclo-[D-Alanyl-D-Valyl-L-Lysyl-L-Lysyl-L-Leucyl-D-Leucine], or a pharmaceutically acceptable salt of any of the foregoing.

6. Use of a cyclic peptide according to any one of claims 1 to 5 in the manufacture of a medicament for use in the treatment of peptic ulcer disease.

7. A method of treatment of peptic ulcer disease, which method comprises the administration of an anti-ulcer effective amount of a cyclic peptide according to any one of claims 1 to 5.